# FASCICULE DE BREVET EUROPEEN

(11) **EP 2 814 557 B1**
(45) Date de publication et mention de la délivrance du brevet: **25.01.2017**
(21) Numéro de dépôt: 13811537.3
(22) Date de dépôt: 19.12.2013
(51) Int. Cl.: A61F 2/82, A61M 27/00

(54) **SONDE ENDO-URETERALE AMELIOREE**
VERBESSERTE HARNLEITERSONDE
IMPROVED URETERAL STENT

(30) Priorité: 19.12.2012 FR 1262293; 07.03.2013 FR 1352061
(43) Date de publication de la demande: 24.12.2014
(73) Titulaire: Vogt, Benoît, 37000 Tours (FR); Desfemmes, François-Noël, 37110 Autrèche (FR); Desgrippes, Arnaud, 41000 Blois (FR)
(72) Inventeur: Vogt, Benoît, 37000 Tours (FR); Desfemmes, François-Noël, 37110 Autrèche (FR); Desgrippes, Arnaud, 41000 Blois (FR)
(74) Mandataire: Regimbeau
(86) Numéro de dépôt international: PCT/EP2013/077506
(87) Numéro de publication internationale: WO 2014/096264

(56) Documents cités:
- EP-A1- 2 308 527
- WO-A1-2005/096991
- DE-A1- 4 103 573
- DE-C1- 3 740 288

## Description

### DOMAINE DE L'INVENTION

Le domaine de l'invention est celui des sondes endo-urétérales, utilisées pour le maintien de l'écoulement urinaire dans l'uretère.

### ETAT DE LA TECHNIQUE

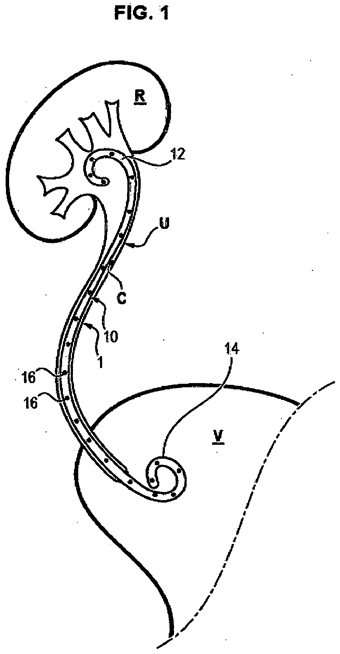

En référence à la figure 1, l'uretère U est un canal reliant un rein R à la vessie V, et dans lequel l'urine coule librement en l'absence d'obstacle.

L'uretère peut se trouver obstrué dans certaines conditions, par exemple en présence de calculs rénaux C, de syndromes de jonction pyélo-urétérale, en cas de compression extrinsèque par fibrose rétro-péritonéale maligne ou bénigne, en cas de tumeur urétérale ou pyélique, ou encore en cas de rétrécissement urétéral ischémique ou radique.

En cas d'obstruction de l'uretère, l'urine ne parvient plus à couler vers la vessie et le rein se dilate. Il survient alors une crise de colique néphrétique.

Pour remédier à cette obstruction, il est connu d'utiliser une sonde endo-urétérale 1 tubulaire, présentant une portion médiane 10 placée dans l'uretère, cette portion s'étendant entre deux extrémités 12, 14.

Une première extrémité 12 débouche dans le rein, et une deuxième extrémité 14 débouche dans la vessie, ces deux extrémités étant recourbées pour assurer le maintien en position de la sonde. Du fait que les deux extrémités sont recourbées en forme de J, une telle sonde est communément appelée « sonde JJ ».

Les sondes JJ sont en général en polyuréthane, en silicone ou en métal et présentent un diamètre moyen de 6 ou 7 French, soit 2 ou 2,3 mm. Elles sont posées pour une durée de 6 à 12 mois.

La sonde 1 comprend sur toute sa longueur une pluralité d'orifices traversants 16, permettant la communication de fluide, en l'espèce d'urine, entre le canal interne de la sonde et l'environnement dans lequel elle est placée.

Ainsi, après la pose de la sonde, l'urine peut de nouveau couler librement entre le rein et la vessie au travers de la sonde 1 et des orifices 16. Elle peut s'écouler uniquement via le canal interne de la sonde ou entre les parois de la sonde et l'uretère en fonction de la nature et de la position de l'obstacle dans l'uretère.

Les sondes JJ ont déjà rendu de grands services, mais elles présentent néanmoins certains inconvénients.

D'une part, après la pose de cette sonde, l'urine peut non seulement couler librement depuis le rein vers la vessie, mais également en sens inverse depuis la vessie vers le rein, car la sonde ouvre un système anatomique qui empêche normalement l'urine de refluer de la vessie vers le rein.

Cette remontée anormale d'urine vers le rein est désagréable et douloureuse.

En outre, cette sonde, du fait de son diamètre peut frotter dans la vessie ou contre l'uretère, et donner des irritations, oedèmes, des pesanteurs pelviennes, ou encore des envies de miction impérieuses chez l'homme comme chez la femme.

Pour toutes ces raisons, la sonde est mal supportée par environ 80 % des patients.

Pour remédier à ce désagrément, il a été proposé dans le document EP2308527 une sonde endo-urétérale dont la partie d'extrémité vésicale en J a été remplacée par au moins deux fils reliés à leurs extrémités de manière à former des boucles de fil. Chaque fil présente un diamètre supérieur à 0.5 French, c'est-à-dire supérieur 0.16 mm, et de préférence compris entre 2 et 4 French, c'est-à-dire entre 0,66 et 1,3 mm, de manière à obtenir, avec les deux fils recourbés, une section suffisamment importante pour permettre un bon drainage de l'urine. Par ailleurs, la queue est décrite comme généralement rigide pour garder la forme en mémoire.

Il est en effet considéré comme impératif de conserver une épaisseur de fil suffisamment importante pour permettre le drainage de l'urine, afin d'éviter le risque de ne pas drainer les urines et d'aboutir à une pyélonéphrite obstructive, voire à un choc septique iatrogène.

Pour autant, la sonde proposée par ce document ne résout pas les problématiques décrites ci-avant car elle engendre toujours des irritations.

Il a été également proposé dans le document WO 2005/096991 une sonde dont une portion d'extrémité vésicale est remplacée par un unique fil.

Le document DE 3740288 décrit un enseignement analogue, avec une sonde comporte une portion d'extrémité rénale de laquelle partent deux fils noués ensemble pour former une portion d'extrémité vésicale filaire.

Le document DE 41 03 573 décrit également une sonde comprenant un fil attaché à une portion tubulaire.

Dans tous ces documents, la portion tubulaire présente une section nette et donc une transition abrupte avec la partie filaire susceptible de générer des irritations importantes dans la partie filaire.

### PRESENTATION DE L'INVENTION

L'invention a pour but de pallier les problèmes mentionnés ci-avant. En particulier, l'invention a pour but de proposer une sonde endo-urétérale permettant le drainage de l'urine du rein à la vessie, sans occasionner d'irritations chez le patient ni de reflux.

A cet égard, l'invention, a pour objet une sonde endo-urétérale selon la revendication 1.

Avantageusement, mais facultativement, la sonde selon l'invention peut en outre comprendre au moins l'une des caractéristiques suivantes :
- Le ou les fils sont de section circulaire, chaque fil présentant un diamètre inférieur à 0,15 mm.
- la partie d'extrémité vésicale comporte un unique fil.
- la portion intermédiaire présente une longueur de 3 cm.
- le diamètre de la portion intermédiaire diminue de façon régulière sur toute la longueur de ladite portion.
- La portion intermédiaire est biseautée.
- la portion intermédiaire comprend une portion tubulaire et une portion pleine prolongée par la portion d'extrémité vésical, le fil de la portion d'extrémité vésicale étant venu de matière avec une paroi de la portion intermédiaire.
- La partie d'extrémité vésicale présente une longueur supérieure ou égale à 2 cm, de préférence comprise entre 5 et 30 cm.
- la partie d'extrémité rénale et la portion intermédiaire comprennent chacune une pluralité de perforations traversantes.
- La sonde comprend en outre une portion urétérale tubulaire disposée entre la partie d'extrémité rénale et la portion intermédiaire, ladite portion urétérale présentant un diamètre externe égal à celui de la partie d'extrémité rénale et étant dépourvue de perforations.
- La portion urétérale tubulaire présente une longueur comprise entre 10 et 25 cm.

Un procédé de traitement d'un trouble urologique par l'implantation d'une sonde endo-urétérale est décrit à titre d'exemple, comprenant :
- l'implantation d'une sonde endo-urétérale dans l'uretère d'un sujet, la sonde endo-urétérale comprenant une portion d'extrémité rénale, une portion d'extrémité vésicale, et une portion intermédiaire s'étendant entre la portion d'extrémité rénale et la portion d'extrémité vésicale, la portion intermédiaire ayant un diamètre externe décroissant vers la portion d'extrémité vésicale, et la portion d'extrémité vésicale comprenant au moins un fil s'étendant depuis la portion intermédiaire, et
- le traitement d'un trouble urologique par la dilation de l'uretère avec la sonde endo-urétérale.

Dans certains cas, le procédé de traitement d'un trouble urologique peut en outre comprendre au moins l'une des caractéristiques suivantes :
- l'implantation de la sonde comprend le positionnement de la partie d'extrémité rénale dans un rein d'un sujet et du au moins un fil dans l'uretère de sorte que ledit au moins un fil cause la dilatation de l'uretère.
- L'implantation de la sonde comprend le positionnement de la portion d'extrémité rénale dans le rein et la portion intermédiaire dans l'uretère de sorte que ledit au moins un fil s'étende depuis l'uretère jusque dans la vessie.
- L'implantation comprend le positionnement de la portion intermédiaire dans le rein de sorte que ledit au moins un fil s'étend depuis le rein dans l'uretère.
- Le trouble urologique comprend des calculs rénaux ou urétéraux, et la dilatation de l'uretère comprend la dilatation de l'uretère avec ledit au moins un fil à un diamètre qui autorise les calculs, ou des fragments desdits calculs, à être évacués naturellement durant la miction.
- Le trouble urologique comprend des calculs rénaux ou urétéraux, syndrome obstructif de la jonction pyélo-urétérale, sténose urétérale tumorale extrinsèque, fibrose rétro-péritonéale maligne, fibrose rétro-péritonéale bénigne, une sténose urétérale ischémique, sténose urétérale post-radique, ou sténose urétérale ou pyélique tumorale intrinsèque.

Une méthode de traitement d'un trouble urologique par l'implantation d'une sonde endo-urétérale est décrite à titre d'exemple, la méthode comprenant :
- L'implantation d'une sonde endo-urétérale dans l'uretère d'un sujet, la sonde endo-urétérale ayant une portion filaire dimensionnée pour dilater l'uretère, et
- Le traitement du trouble urologique par la dilatation de l'uretère avec la partie filaire de la sonde endo-uretérale.

Dans certains cas, cette méthode de traitement comprend en outre au moins l'une des caractéristiques suivantes :
- La partie filaire comprend une section circulaire de diamètre inférieur à 0.15 mm.
- La sonde endo-urétérale comprend en outre une portion tubulaire ayant un diamètre décroissant vers une première extrémité, et dans laquelle la partie filaire s'étend depuis ladite extrémité de la portion tubulaire, et l'implantation comprend le positionnement de la portion tubulaire dans un rein d'un sujet et la partie filaire dans l'uretère.
- La portion tubulaire comprend une partie recourbée à une extrémité opposée de la première extrémité, et la partie recourbée et la première extrémité comprennent des orifices, et une portion urétérale s'étendant entre la partie recourbée et la première extrémité est dépourvue d'orifices, et l'implantation comprend le positionnement de la portion urétérale et de la partie filaire dans l'uretère.
- Le trouble urologique comprend des calculs rénaux ou des calculs urétéraux, et le traitement comprend l'évacuation des calculs, ou de fragments des calculs, du rein ou de l'uretère lorsque la sonde endo-urétérale reste implantée dans l'uretère.
- Le trouble urologique comprend l'un parmi des calculs rénaux, calculs urétéraux, ou syndrome obstructif de a jonction pyélo-urétérale, et l'implantation comprend le positionnement de la portion tubulaire de la sonde endo-urétérale dans un rein d'un sujet et la partie filaire dans l'uretère.
- Le trouble urologique comprend l'un d'une sténose urétérale tumorale extrinsèque, fibrose rétro-péritonéale maligne, fibrose rétro-péritonéale bénigne, une sténose urétérale ischémique, sténose urétérale post-radique, ou sténose urétérale ou pyélique tumorale intrinsèque.

De manière surprenante, et à l'encontre des préjugés établis chez les praticiens, le faible diamètre de la partie d'extrémité vésicale est suffisant pour permettre le drainage de l'urine jusqu'à la vessie. De plus, cette partie engendre peu voire pas d'irritations ou d'inconfort chez le patient chez qui la sonde a été implantée.

De manière plus surprenante encore, il a été constaté que la pose d'une telle sonde engendre une dilatation du méat et de l'uretère par le fil de la partie d'extrémité vésicale. Cette dilatation du méat permet l'introduction aisée d'un urétéroscope rigide (d'un diamètre de l'ordre de 4 à 5 mm) dans l'uretère ou d'une gaine pour urétéroscopie souple, d'un diamètre de l'ordre de 3 à 4 mm.

En outre, les fragments de calculs sont évacués autour du ou des fils et la partie d'extrémité rénale sans aucune douleur de colique néphrétique. Ce phénomène est expliqué par le fait que la dilatation de l'uretère favorise l'évacuation des fragments, et le ou les fils empêchent l'obstruction brutale.

Enfin, la portion intermédiaire de diamètre décroissant entre les deux parties d'extrémité étant régulièrement effilée, elle diminue le risque d'oedème pouvant résulter d'un accrochage de la sonde dans une portion d'uretère.

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :
- La figure 1, déjà décrite, représente schématiquement l'implantation d'une sonde de l'état de la technique dans un uretère comprenant un calcul rénal.
- La figure 2a représente schématiquement une sonde endo-urétérale conforme à un mode de réalisation de l'invention,
- La figure 2b représente schématiquement l'implantation d'une sonde représentée en figure 2a dans un uretère comprenant un calcul urétéral.

Les figures 2c et 2d représentent deux variantes de la sonde de la figure 2a.
- La figure 3a représente schématiquement une sonde endo-urétérale conforme à un deuxième mode de réalisation de l'invention.
- La figure 3b représente schématiquement l'implantation d'une sonde de la figure 3a dans un uretère.
- La figure 4a est un scanner réalisé sur un patient dans lequel une sonde a été implantée, illustrant la dilatation de l'uretère consécutive à la pose de la sonde.
- La figure 4b est une photographie prise de l'intérieur d'un uretère dans lequel une sonde a été mise en place, et qui illustre également la dilatation de l'uretère.
- Les figures 5a à 5c sont des radiographies représentant l'évacuation naturelle de calcul grâce à la mise en place d'une sonde.
- Les figures 6a et 6b représentent des vues en coupe longitudinale d'une portion intermédiaire d'une sonde.
- Les figures 7a à 7c illustrent schématiquement le mouvement d'une sonde dans un uretère lors d'une respiration d'un individu.
- La figure 8a représente schématiquement une sonde endo-urétérale selon une variante de réalisation de celle de la figure 2a.
- La figure 8b représente schématiquement l'implantation d'une sonde de la figure 8a dans un uretère comprimé par une tumeur extrinsèque.

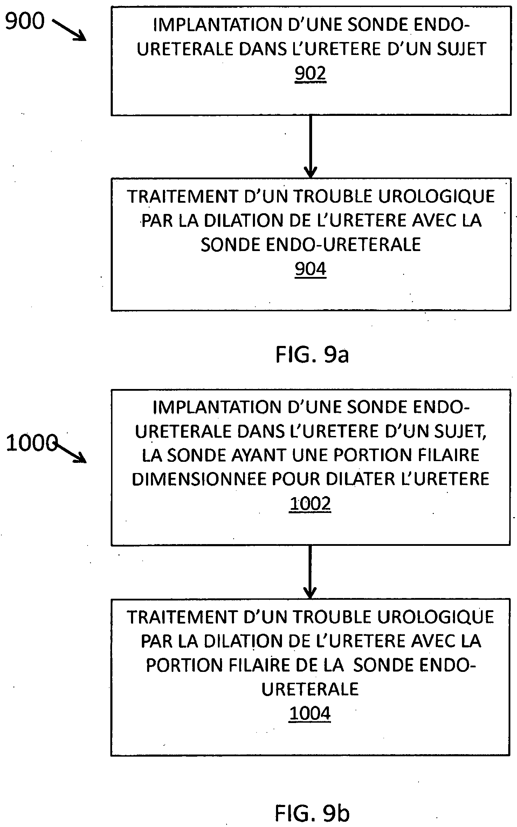

- Les figures 9a et 9b représentent schématiquement des étapes d'un traitement thérapeutique mis en oeuvre avec la sonde.

### DESCRIPTION DETAILLEE D'AU MOINS UN MODE DE REALISATION DE L'INVENTION

Dans toute la suite, seuls les modes de réalisation prévoyant un raccordement de la partie d'extrémité vésicale à la portion intermédiaire conforme à la revendication 1 font partie de l'invention.

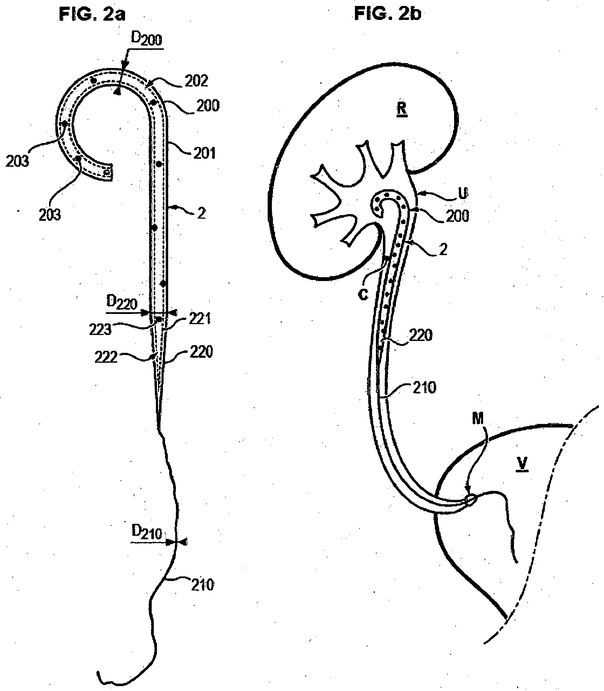

En référence à la figure 2a, on a représenté un mode de réalisation d'une sonde endo-urétérale.

Cette sonde 2 comporte une première partie d'extrémité rénale 200. Cette partie est tubulaire; c'est-à-dire qu'elle comprend une paroi cylindrique 201 délimitant un canal interne 202 dans lequel un fluide peut circuler. Une pluralité d'orifices traversants 203 sont en outre agencés dans la paroi cylindrique 201, permettant au fluide de pénétrer dans le canal interne 202 ou d'en sortie au travers de la paroi 201.

La partie 200 d'extrémité rénale est recourbée en forme de J, c'est-à-dire qu'elle présente une extrémité en demi-cercle ou en forme de boucle (c'est-à-dire en forme d'anneau) permettant, une fois que la sonde est implantée, le maintien de cette partie 200 dans un rein R.

Alternativement, cette partie peut présenter un ou plusieurs segments reliés entre eux par des coudes arrondis pour éviter toute section brusque ou coupante.

En référence à la figure 2b, la partie 200 d'extrémité rénale est configurée pour pénétrer dans le rein une fois la sonde en place, et s'étendre depuis le rein R vers l'uretère U du patient. A cet égard, elle présente une longueur, lorsqu'elle est recourbée, comprise entre 6 et 20 cm, de préférence entre 8 et 10 cm, avantageusement égale à 9 cm. Ceci correspond à une longueur totale, en maintenant cette partie droite, comprise entre 12 et 26 cm, de préférence entre 14 et 16 cm, et avantageusement de l'ordre de 15 cm. Cette variation de longueur dépend de la localisation de l'obstacle au niveau de l'uretère car en effet la partie d'extrémité rénale 200 "court-circuite" avantageusement l'obstacle, c'est à dire se termine à un niveau de l'urétère ne comprenant plus d'obstacle.

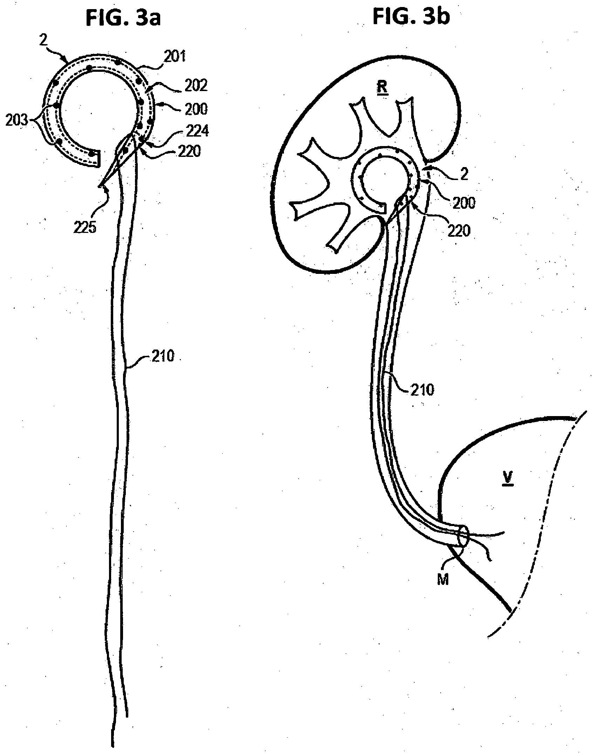

Selon une variante de réalisation représentée en figures 3a et 3b, cette partie d'extrémité rénale est limitée à la boucle du « J », c'est-à-dire à un unique anneau tubulaire, de l'ordre de 22 mm de diamètre lorsque la boucle est recourbée, ce qui correspond à une longueur, en maintenant cette partie droite, de l'ordre de 7 cm, ou plus généralement un diamètre compris entre 10 et 25 mm, le diamètre minimal servant à empêcher la boucle de pénétrer dans l'uretère en état recourbé, et le diamètre maximal servant étant limité pour ne pas causer de douleur ou de gêne.

De retour au mode de réalisation de la figure 2a, la partie 200 présente un diamètre externe D₂₀₁ constant, compris entre 2 et 3,4 mm (soit de 6 à 10 French), de préférence compris entre 2 et 2.4 mm (soit de 6 à 7 French), et très avantageusement de l'ordre de 2.4 mm (soit 7 French). En revanche, dans le mode de réalisation des figures 3a et 3b, cette partie 200 présente préférablement un diamètre externe constant compris entre 1 et 3 mm, et de préférence de l'ordre de 2 mm (soit 6 French), voire de 1.6 mm (soit 4,8 French) pour que cette partie, plus courte, soit aussi plus souple pour permettre de la déplier lors de son retrait.

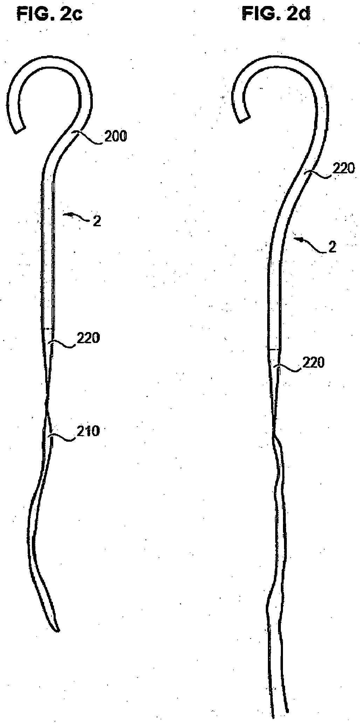

Sur les deux modes de réalisation des figures 2a, 2b et 3a et 3b, la sonde 2 présente en outre une deuxième partie d'extrémité 210 vésicale, destinée à pénétrer dans la vessie V, et à s'étendre depuis l'uretère U jusque dans la vessie V. Cette partie comprend au moins un fil, et de préférence peut comprendre un fil unique, qui peut être libre ou être replié pour former une boucle, comme illustré en figure 2c, ou deux fils, illustrés en figure 2d, de préférence pleins, c'est-à-dire de section circulaire et dépourvus de canal interne. L'absence de canal interne supprime le risque de reflux de l'urine vers le rein.

En référence à la figure 2b, chaque fil présente un diamètre D₂₁₀ constant inférieur à 0,15 mm, c'est-à-dire 0.45 French, de préférence compris entre 0,05 et 0,15 mm, encore plus avantageusement compris entre 0,1 et 0,15 mm, c'est-à-dire de 0,3 à 0,45 French, et de préférence égal à 0,1mm ou 0,3 French. En tout état de cause, le diamètre du fil ou la section cumulée des fils ne doit pas excéder 0,3 mm.

Bien que l'emploi d'un fil unique soit suffisant pour drainer l'urine, l'utilisation d'une boucle de fils peut être plus rassurante en cas de rupture de l'un des deux.

La séparation en deux fils évite aussi la rigidité d'un fil unique plus épais.

Les inventeurs ont découvert que, de manière surprenante, l'utilisation d'un seul fil de faible diamètre permet, contrairement aux préjugés répandus dans le domaine, de drainer l'urine depuis l'uretère vers la vessie.

En effet, de manière surprenante, la sonde proposée, et notamment le fil de faible diamètre, entraine une dilatation de l'uretère, dans un délai de deux semaines à dix jours après la pose de la sonde.

De manière encore plus surprenante, la pose d'une sonde, et notamment d'une sonde conforme au mode de réalisation des figures 3a et 3b, c'est-à-dire lorsque la portion d'extrémité rénale de la sonde est limitée à une boucle disposée dans le rein, entraine aussi, dans environ 50% des cas, une dilatation des cavités rénales (bassinet et calices). Ces dilatations n'entrainent aucune douleur.

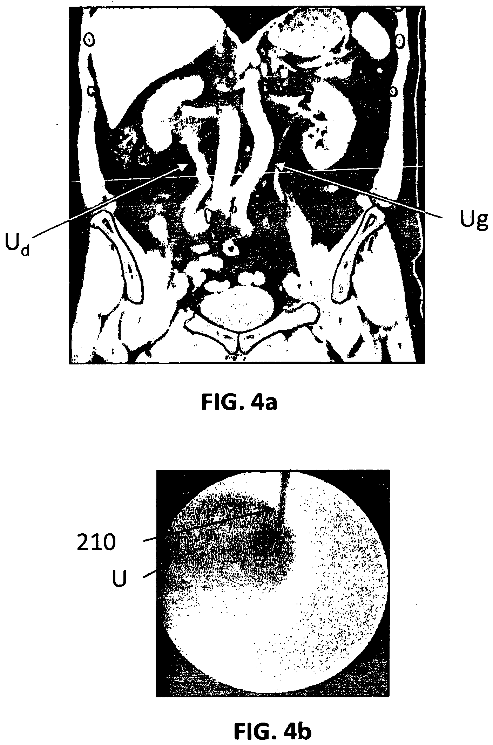

La dilatation de l'uretère est visible sur la figure 4a, qui est un scanner réalisé chez un patient dans lequel une sonde a été placée dans l'uretère droit, l'autre uretère étant dépourvu de sonde. L'uretère droit Ud, à gauche sur la figure, apparaît fortement dilaté, notamment en comparaison avec l'uretère gauche Ug se trouvant à droite.

La dilatation de l'uretère est également visible dans la figure 4b, qui est une photographie de l'intérieur de l'uretère prise en endoscopie. On remarque le fil 210 de la sonde.

Des mesures statistiques de dilatation de l'uretère ont été réalisées sur des patients comprenant seulement une sonde, en comparant les diamètres de l'uretère et du bassinet du rein du côté de la sonde aux mêmes diamètres sur le côté sans sonde.

On a appelé Sondé S1 une sonde conforme au mode de réalisation des figures 2a et 2b, et Sonde S2 une sonde conforme au mode de réalisation des figures 3a et 3b, dans lequel la partie rénale est plus courte et la portion intermédiaire 220 se trouve aussi dans le rein.

Les résultats des mesures de diamètres sont représentés dans la table 1.

**Table 1**

| | Côté Sonde S1 | Côté contro-latéral | *p* | Côté Sonde S2 | Conté contro-latéral | *p* |
|---|---|---|---|---|---|---|
| Nombre de scanners analysés | 17 | | | 13 | | |
| Délai entre pose et scanner (jours) | 43.4±34. 1 | | | 49.4±22 | | |
| Dilatation des cavités | 31.3% | | | 50.0% | | |
| Bassinet (mm) | 17.8±4.9 | 5.1±1.4 | 0.003 | 17.4±8.3 | 7.8±4.7 | 0.003 |
| Uretère lombaire (mm) | 8.7±1.7 | 3.1±0.5 | 0.00000001 | 9.7±2.9 | 4.2±1.6 | 0.00005 |
| Uretère iliaque (mm) | 65±1.3 | | 0.000006 | 8.1±1.6 | 3.2±0.9 | 0.000001 |
| Uretère pelvien (mm) | 6.5±1.4 | 3.8±0.8 | 0.000001 | 6.4±2.0 | 3.3±0.8 | 0.0004 |

Une telle dilatation provoque un autre effet imprévu : celui de l'évacuation naturelle de fragments de calculs présents dans le rein ou dans l'uretère.

Ceci s'applique dans le cas de fragments de calculs (ou calculs entiers) glissants sans oedème dans un uretère sain.

Par conséquent, il est proposé une méthode de traitement de calculs rénaux. Une première étape consiste à implanter une sonde dans l'uretère et le rein. Dans le cas où un calcul est présent sans toutefois entraîner d'obstruction de l'uretère, la sonde utilisée est de préférence conforme au mode de réalisation des figures 3a et 3b, car il n'est pas nécessaire de drainer l'urine par l'intérieur de la sonde pour contourner un obstacle.

Alternativement, dans le cas d'une obstruction de l'uretère, on peut employer une sonde dont la portion intermédiaire se trouve dans l'uretère, conformément au mode de réalisation des figures 2a et 2b, pour drainer l'urine au-delà de l'obstacle par l'intérieur de la sonde.

Après la pose de la sonde, l'uretère est dilaté sur toute sa longueur, et en outre le ou les fils de la sonde sont suffisamment fins pour ne pas encombrer l'uretère et permettent l'insertion d'un urétéroscope associé à un outil ad hoc, par exemple une fibre laser, pour traiter localement un calcul. Il n'est donc pas nécessaire, contrairement aux interventions mises en oeuvre auparavant après la mise en place d'une sonde JJ, de retirer la sonde JJ pour traiter le calcul, puis de remettre une sonde en place. Il en résulte une diminution des manipulations et du coût de l'intervention.

Après la pose de la sonde, le traitement diffère ensuite en fonction de la taille du calcul à évacuer. Pour de petits calculs, de taille inférieure à environ 10 mm, ces calculs peuvent être évacués naturellement de l'uretère vers la vessie par le flux d'urine dans l'uretère, puis de la vessie au dehors par la miction.

Dans le cas de calculs de taille plus importante, par exemple supérieure à 10 mm, une seconde étape consiste à fragmenter le calcul.

Cette étape est de préférence mise en oeuvre un certain temps après la mise en place de la sonde, pour que l'uretère soit suffisamment dilaté. Ainsi il est préférable d'attendre un délai de dix jours après la pose de la sonde, et de préférence de l'ordre de quinze jours à trois semaines pour fragmenter le calcul.

Cette fragmentation peut être réalisée selon des méthodes connues par lithotritie extracorporelle, ou par urétéroscopie (ondes de choc ou laser) ou par néphroscopie percutanée (ondes de choc ou laser).

Les calculs ou fragments glissent ensuite du rein vers l'uretère, puis de l'uretère dans la vessie et enfin hors de la vessie sans douleur.

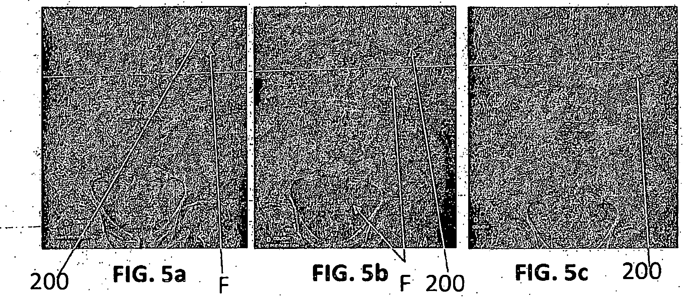

Les figures 5a à 5c sont des radiographies d'un patient à différents stades d'évacuation de fragments d'un calcul rénal. Le jour de la prise de la radiographie de la figure 5a, une sonde 2 conforme au mode de réalisation des figures 3a et 3b a été positionnée dans l'uretère 15 jours auparavant, et le calcul a été fractionné en fragments F.

La radiographie de la figure 5b a été réalisée trente jours après la mise en place de la sonde, et l'on peut y observer le déplacement des fragments F.

La radiographie de la figure 5c a été réalisée 42 jours après la mise en place de la sonde, et l'on remarque que tous les fragments ont été évacués.

Il a été constaté par les inventeurs que les dilatations observées disparaissent après le retrait de la sonde.

En outre, du fait de son diamètre limité, le fil est très flexible et ne cause aucune irritation, les patients sont donc soulagés et voient leur confort de vie s'améliorer.

Enfin, et contrairement aux sondes précédemment utilisées, qui étaient systématiquement calcifiées, il a été constaté avec surprise par les inventeurs qu'aucune calcification n'apparaît sur le ou les fils 210, jusqu'à au moins six mois après la pose de la sonde.

La diminution d'une gêne consécutive à la pose d'une sonde a été quantifiée chez un groupe de patients. Cette amélioration concerne la tolérance vésicale et est certainement due à la finesse extrême du fil 210.

La table 2 récapitule des symptômes urinaires ressentis par des patients chez qui une sonde JJ mal tolérée a été remplacée par une Sonde S1 (figures 2a et 2b). On a évalué par questionnaire la tolérance du fil 210 par rapport à la tolérance de la partie vésicale de la sonde JJ.

Chaque question présentait cinq réponses possibles, auxquelles des scores de 1 à 5 ont été attribués, 1 étant le score attribué à la réponse correspondant à une gêne nulle et 5 à la gêne la plus importante.

Par exemple, pour une question portant sur la fréquence de fuites urinaires, le score 1 est attribué à la réponse « Jamais », 2 à la réponse « Rarement », 3 à « Parfois », 4 à « La plupart du temps », et 5 à « Tout le temps ».

La table 2 indique les scores moyens ressentis par les patients.

**Table 2**

| Groupe de patients (N=24) | Sonde JJ | Sonde S1 après JJ | p |
|---|---|---|---|
| Symptômes urinaires | | | |
| Pollakiurie | 3.7 ± 1.3 | 2.8 ± 1.1 | 0.005 |
| Nycturie | 3.8 ± 1.1 | 2.8 ± 1.2 | 0.0001 |
| Urgences | 3.2 ± 1.2 | 2.4 ± 1.0 | 0.01 |
| Urgences non retenues | 2.0 ± 0.9 | 1.8 ± 0.8 | 0.46 |
| Incontinence non urgente | 1.8 ± .09 | 1.1 ± 0.3 | 0.004 |
| Sensation de résidu | 3.0 ± 1.2 | 1.8 ± 0.9 | 0.00003 |
| Brûlures urétrales | 3.9 ± 1.3 | 1.8 ± 1.1 | 0.00000005 |
| Hématurie | 2.8 ± 1.4 | 1.6 ± 0.8 | 0.0006 |
| Aspect de l'urine | 2.1 ± 0.8 | 1.6 ± 0.8 | 0.01 |
| Retentissement social | 3.8 ± 1.0 | 2.3 ± 0.9 | 0.00003 |
| Impact sur la qualité de vie | 5.3 ± 2.0 | 3.8 ± 1.6 | 0.0007 |
| Score total | 35.2 ± 7.5 | 23.6 ± 5.4 | 0.000002 |

De retour à la figure 2a, la partie 210 d'extrémité vésicale présente une longueur supérieure ou égale à 2 cm, de préférence comprise entre 5 et 30 cm, de préférence comprise entre 20 cm et 28 cm.

A cet égard, si la partie d'extrémité vésicale est constituée d'un fil replié, par exemple en son milieu, pour former un double-fil, le fil utilisé mesure donc le double de la partie d'extrémité vésicale, soit au moins 4 cm, et de préférence entre 10 et 60 cm.

La partie du fil pénétrant à l'intérieur de la vessie V après mise en place de la sonde 2 est laissée libre dans la vessie et peut être recoupée si besoin.

Dans le cas où le fil est entraîné par le flux urinaire, la partie au-dessous du sphincter n'a pas traduction clinique. En particulier, il n'y a pas d'incontinence.

En outre, les inventeurs se sont aperçus que la transition entre la partie d'extrémité rénale 200 et la partie d'extrémité vésicale 210 doit être progressive pour éviter qu'un changement brusque de diamètre de la sonde ne s'accroche dans des coudes de l'uretère U.

A cet égard, la sonde comporte en outre une portion intermédiaire 220, s'étendant entre la partie d'extrémité rénale 200 et la partie d'extrémité vésicale 210.

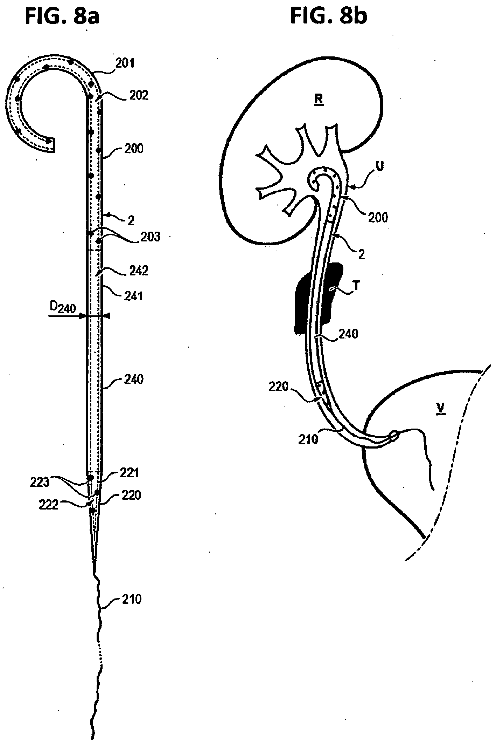

Selon le premier mode de réalisation, sur les figures 2a à 2d et 8a et 8b, cette portion intermédiaire est destinée à être positionnée dans l'uretère U lorsque la sonde 2 est en place.

Selon le mode de réalisation alternatif représenté en figures 3a et 3b, la portion intermédiaire est destinée à être positionnée dans le rein R lorsque la sonde 2 est en place, de sorte qu'il ne se trouve qu'un ou deux fils dans l'uretère.

Cette portion intermédiaire 220 est tubulaire, c'est-à-dire qu'elle comprend une paroi 221 de section annulaire et comprend en outre un canal interne 222 dans lequel un guide peut coulisser pour la mise en place de la sonde, et dans lequel un fluide peut circuler une fois la sonde en place.

Le canal 222 se situe dans le prolongement du canal 202 de la partie d'extrémité rénale de manière à autoriser la communication de fluide entre les deux.

En référence aux figures 2a, et 2b, la portion intermédiaire 220 présente un diamètre externe D₂₂₀ variable, décroissant vers la partie d'extrémité vésicale 210 de sorte que le diamètre externe minimal de la portion intermédiaire 220 au niveau du raccordement avec le fil soit sensiblement égal au diamètre D₂₁₀ du fil de la partie d'extrémité vésicale 210. Par sensiblement, on entend que le diamètre minimal de la portion intermédiaire 220 ne doit pas excéder deux fois le diamètre du fil, ou la section cumulée des fils s'il y en a plusieurs, et en tout état de cause doit être strictement inférieur à 1 mm, et de préférence inférieur à 0.5 mm, le raccordement entre la portion intermédiaire et le ou les fils étant alors lissé au maximum (par exemple chanfreiné) pour éviter toute aspérité en dent de scie susceptible de causer une douleur. Ceci permet d'assurer une transition douce et régulière entre les deux diamètres extrêmes de la sonde et de conférer plus de souplesse à la sonde pour que celle-ci puisse s'adapter à la courbure de l'uretère. Ceci diminue les douleurs liées à la présence de la sonde et facilite son retrait.

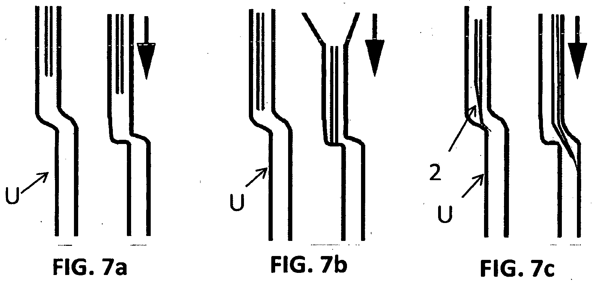

On a en effet représenté, dans les figures 7a à 7c, les déplacements de la sonde dans l'uretère avec la respiration de l'individu dans lequel la sonde est implantée. On distingue les 3 cas suivants :
Sur la figure 7a, une sonde du type JJ présente une coupure nette sans portion intermédiaire 220 assurant une transition entre les deux. Si la sonde se trouve à distance d'une sinuosité de l'uretère, les mouvements de cette sonde n'entrainent pas de douleur.

En revanche, en référence à la figure 7b, si cette sonde se déplace dans l'uretère et parvient au niveau d'une sinuosité, les mouvements de cette sonde dans l'uretère avec la respiration provoquent des douleurs du fait que l'extrémité abrupte et rigide de la sonde s'enfonce contre la paroi de l'uretère.

En référence à la figure 7c, on constate que la présence d'une potion intermédiaire formant la transition entre le fil et la partie d'extrémité rénale permet d'assouplir la sonde et donc de supprimer les douleurs dans les sinuosités de l'uretère lors de la respiration.

De la même manière, le retrait de la sonde est facilité, puisque la portion intermédiaire confère à la sonde une souplesse qui permet de guider cette sonde dans l'uretère lors de son retrait sans la bloquer.

Le diamètre externe maximal de la portion intermédiaire 220 est égal à celui de la partie d'extrémité rénale.

Ainsi, à titre d'exemple non limitatif, la portion intermédiaire présente un diamètre décroissant de façon régulière depuis un diamètre d'environ 2.4 mm à un diamètre d'environ 0.15 mm.

La partie tubulaire de la sonde 2, c'est-à-dire la partie de la sonde qui comprend un canal interne, se termine au niveau de l'extrémité de la portion intermédiaire 220.

A cet égard, le canal interne 222 de la portion 220 débouche dans l'uretère au travers d'un orifice (non représenté) pratiqué dans la paroi 221, à proximité de la jonction avec le fil 210 de la partie d'extrémité vésicale.

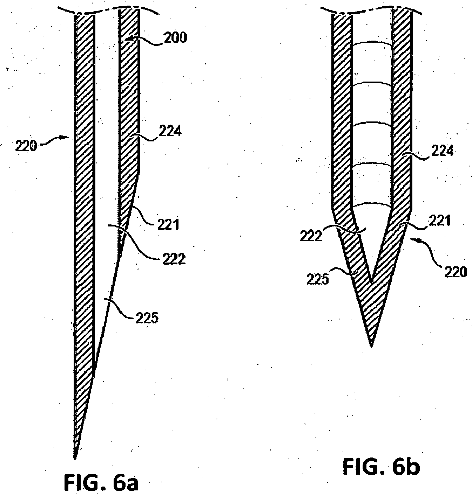

Selon un mode de réalisation préféré, représenté en figures 6a et 6b, la portion intermédiaire 220 de la sonde est taillée en biseau, de sorte que la portion intermédiaire comprend une première portion 224 tubulaire, et une deuxième portion 225 terminant la portion intermédiaire 220, la portion 225 étant ouverte latéralement, de sorte que le canal 222 débouche dans l'uretère, ladite deuxième portion étant de fait pleine, c'est-à-dire non tubulaire, et étant prolongée par la partie d'extrémité vésicale 210. Ce mode de réalisation est avantageux car la forme en biseau présente une continuité qui permet à la sonde de ne pas accrocher dans l'uretère.

En outre, la portion 225, du fait du biseau, est très souple, ce qui diminue encore le risque d'accrochage et guide la sonde au sein des replis urétéraux durant les mouvements de respiration.

Le fil 210 peut être venu de matière avec la paroi de la portion intermédiaire 220. Alternativement, il peut être rapporté sur celle-ci, par exemple collé ou fixé sur la paroi, ou encore noué au niveau d'un orifice 223.

Avantageusement, le fil 210 émerge depuis le canal interne 222 de la portion 220, c'est-à-dire qu'il est solidarisé à la paroi 221 cylindrique au niveau d'une surface intérieure de celle-ci, et ce de façon à ne pas former une excroissance sur la surface extérieure de la paroi 221, comme illustré schématiquement en figure 2a.

Dans le mode de réalisation des figures 3a et 3b, un fil est avantageusement rapporté sur la portion intermédiaire 220 et plié en deux, de préférence en son milieu, pour obtenir un double fil. Cette réalisation est toutefois applicable également au mode de réalisation des figures 2a et 2b.

Selon un exemple non couvert par l'invention, et comme visible sur la figure 3a, le fil traverse un premier orifice de la paroi de ladite portion 220 au niveau de la pointe biseautée 225 de celle-ci, et retraverse la paroi de la sonde au voisinage de la jonction entre la portion intermédiaire 220 et la partie d'extrémité rénale 210, c'est-à-dire soit au niveau de la portion tubulaire 224, soit à l'extrémité de la partie d'extrémité rénale 210.

Ceci permet d'augmenter la solidité de la fixation de la partie d'extrémité vésicale à la portion intermédiaire, notamment au retrait de la sonde, car la portion de la sonde au niveau de la pointe biseautée est fragile et peut se briser.

Ceci permet également de retirer la sonde sans douleur, car attacher le fil uniquement au niveau de la partie d'extrémité rénale ou de sa jonction avec la portion intermédiaire pourrait entrainer que la pointe biseautée se recourbe au moment du retrait de la sonde et, en irritant l'uretère ou le rein, provoque des douleurs.

Enfin, le fil est avantageusement noué de part et d'autre de la paroi de la pointe biseautée 225 pour le maintenir en position.

Alternativement, et préférablement, de manière analogue à la représentation de la figure 2a, au moins un fil 210 peut être venu de matière avec la paroi de la portion intermédiaire 220 ou être rapporté sur celle-ci, par exemple collé ou fixé, et émerger du canal interne 222 de la portion intermédiaire 220 (au niveau de la pointe biseautée 225). Ceci permet de préserver la continuité des différentes parties de la sonde et donc de ne pas susciter de gêne chez le patient.

De manière générale, la paroi de la sonde doit être la plus lisse possible, et dépourvue d'aspérités (pas de variation d'angle brusque, de coupure nette, de rugosité de surface) pour éviter toute irritation de l'uretère et tout inconfort du patient.

Sur la figure 2b, la jonction entre la portion intermédiaire 220 et la partie d'extrémité vésicale 210 se situe très préférablement à distance du méat M de l'uretère lorsque la sonde 2 est en place, c'est-à-dire de l'orifice de l'uretère qui débouche dans la vessie, de sorte que seul le fil 210 franchisse le méat et pénètre dans la vessie V.

A cet égard, la portion intermédiaire 220 présente une longueur comprise entre 0,5 et 12 cm, en fonction de la position d'une obstruction de l'uretère - la portion 220 présente alors une section décroissante de façon régulière sur toute sa longueur, de préférence de l'ordre de 3 cm. Cette longueur permet d'assurer la douceur de la transition entre les deux portions extrêmes de la sonde.

La contribution conjuguée du ou des fils et de la transition douce entre ces fils et la partie d'extrémité rénale 200 permet donc d'améliorer significativement le confort des patients.

Dans le mode de réalisation représente en figures 3a et 3b, la portion intermédiaire présente avantageusement une longueur de l'ordre de 1 cm, permettant que seul un ou deux fils pénètrent dans l'uretère puis dans la vessie au travers du méat M.

Ainsi, lorsque la sonde 2 est en place, l'urine est drainée depuis le rein vers la vessie, en passant par le canal interne 202 de la partie d'extrémité rénale et 222 de la portion intermédiaire 220, ou le long des parois de ces parties, puis en s'écoulant le long du fil 210.

Selon un mode de réalisation particulier, représenté en figures 8a et 8b, la sonde, qui reprend toutes les caractéristiques précédentes, comprend en outre une partie urétérale 240 s'étendant entre la partie d'extrémité rénale 200 et la portion intermédiaire 220.

Cette partie urétérale est tubulaire, et comprend donc une paroi cylindrique 241 définissant un canal interne 242 communiquant à une extrémité avec celui 202 de la partie d'extrémité rénale 200 et à une autre extrémité avec celui 222 de la portion intermédiaire 220 pour assurer la communication de fluide tout le long de la sonde.

Cette partie 240 présente un diamètre externe D₂₄₀ égal à celui de la partie d'extrémité rénale 200, et une longueur comprise entre 10 et 25 cm, et de préférence de l'ordre de 20 cm.

En revanche, cette partie 240 est renforcée et notamment ne comprend pas d'orifices perforants, de sorte que le fluide peut uniquement s'écouler à l'intérieur du canal interne 242.

Cette partie se trouve dans l'uretère lorsque la sonde est en place, et permet de faire circuler l'urine via le canal interne 242 en cas de forte compression externe de l'uretère. Cette situation apparaît typiquement en cas de tumeur T adjacente ou entourant l'uretère. L'absence de perforations permet d'éviter à la tumeur T d'obstruer le canal interne 242.

La sonde 2 est de préférence réalisée en un matériau polymère, de préférence du polyuréthane ou du silicone. Le fil de la partie d'extrémité vésicale peut être réalisé en un tel matériau ou en polypropylène.

On a ainsi proposé une sonde qui ne génère plus d'irritations ni de reflux chez les patients, et qui, contrairement à ce qui aurait pu être redouté, parvient à drainer l'urine jusqu'à la vessie.

La mise en place d'une telle sonde est réalisée pour rétablir ou améliorer le drainage de l'urine dans l'uretère, notamment la mise en place d'une sonde conforme aux figures 2a et 2b pour un calcul rénal ou urétéral obstructif et un syndrome de la jonction pyélourétérale obstructif, également d'une sonde conforme aux figures 3a et 3b pour une préparation de l'uretère en vue de pose de gaine pour urétéroscopie souple ou urétéroscopie rigide ou calcul rénal non obstructif avant traitement du calcul (lithotritie extracorporelle, traitement par urétéroscopie ; traitement par néphroscopie), également d'une sonde conforme aux figures 8a et 8b pour une sténose urétérale tumorale extrinsèque ou une fibrose rétro-péritonéale maligne ou bénigne ou une sténose urétérale ischémique ou sténose urétérale post-radique ou sténose urétérale ou pyélique tumorale intrinsèque.

L'intervention a lieu sous anesthésie générale ou régionale. La portion d'extrémité rénale 200 de la sonde est placée dans le rein au travers d'un endoscope et sous amplificateur de brillance. Un poussoir d'environ 50 cm est nécessaire pour pouvoir pousser cette partie jusque dans le rein. Pour pallier le manque de longueur des poussoirs conventionnellement utilisés pour pousser des sondes JJ (comme la portion tubulaire de la sonde 2 selon l'invention est plus courte), on peut par exemple utiliser une sonde urétérale comme la sonde Open-End Flexi-Tip Ureteral Catheter, 5 F (soit 1.67 mm) / 70 cm, Cook Medical.

Le ou les fils de la partie d'extrémité vésicale sont abandonnés dans la vessie en veillant à ne pas les pousser dans l'uretère avec le reste de la sonde.

Pour le retrait, la sonde peut être retirée sous anesthésie locale à l'aide d'un fibroscope et d'une pince en tirant sur un fil. Un fil ne casse pas lors du retrait de la sonde, mais l'ablation peut être délicate faute de matériel de préhension adapté. Par exemple, on peut avantageusement se servir d'une pince à biopsie comme le modèle Karl Storz - Endoskope, Biopsy Forceps, double action jaws, 7F (soit 2,33 mm), longueur 40 cm, 27175A.

## Revendications

1. Sonde endo-urétérale (2), comprenant une partie d'extrémité rénale (200) et une partie d'extrémité vésicale (210), la partie d'extrémité rénale (200) étant tubulaire et recourbée,
ladite sonde (2) étant **caractérisée en ce que** la partie d'extrémité vésicale (210) comporte au moins un fil, et **en ce que** la sonde comprend en outre une portion intermédiaire tubulaire (220) s'étendant entre la partie d'extrémité rénale et la partie d'extrémité vésicale, ladite portion présentant un diamètre externe (D₂₂₀) décroissant vers la partie d'extrémité vésicale, et le diamètre externe minimal de ladite portion (220), au niveau du raccordement avec le fil de la partie d'extrémité vésicale (210) est inférieur à deux fois le diamètre du fil, ou la section cumulée des fils s'il y en a plusieurs, et en tout état de cause est strictement inférieur à 1 mm, ladite portion intermédiaire (220) présentant une longueur comprise entre 0.5 cm et 12 cm.

2. Sonde endo-urétérale (2) selon la revendication 1, dans laquelle le ou les fils sont de section circulaire, chaque fil présentant un diamètre (D₂₁₀) inférieur à 0,15 mm.

3. Sonde endo-urétérale (2) selon la revendication 1 ou 2, dans laquelle la partie d'extrémité vésicale (210) comporte un unique fil.

4. Sonde endo-urétérale selon l'une des revendications précédentes, dans laquelle la portion intermédiaire (220) présente une longueur de 3 cm.

5. Sonde endo-urétérale selon l'une des revendications précédentes, dans laquelle le diamètre de la portion intermédiaire (220) diminue de façon régulière sur toute la longueur de ladite portion.

6. Sonde endo-urétérale (2) selon l'une des revendications précédentes, dans laquelle la portion intermédiaire (220) est biseautée. ,

7. Sonde endo-urétérale (2) selon l'une des revendications 3 à 6, dans laquelle la portion intermédiaire (220) comprend une portion tubulaire (224) et une portion pleine (225) prolongée par la portion d'extrémité vésical (210), le fil de la portion d'extrémité vésicale (210) étant venu de matière avec une paroi de la portion intermédiaire (220).

8. Sonde endo-urétérale (2) selon l'une des revendications précédentes, dans laquelle la partie d'extrémité vésicale (210) présente une longueur supérieure ou égaie à 2 cm, de préférence comprise entre 5 et 30 cm.

9. Sonde endo-urétérale (2) selon l'une des revendications 1 à 8, dans laquelle la partie d'extrémité rénale (200) et la portion intermédiaire (220) comprennent chacune une pluralité de perforations traversantes (203, 223).

10. Sonde endo-urétérale (2) selon l'une des revendications 1 à 9, comprenant en outre une partie urétérale tubulaire (240) disposée entre la partie d'extrémité rénale (200) et la portion intermédiaire (220), ladite partie urétérale (240) présentant un diamètre externe (D₂₄₀) égal à celui de la partie d'extrémité rénale (200) et étant dépourvue de perforations.

11. Sonde endo-urétérale (2) selon la revendication précédente, dans laquelle la partie urétérale (240) tubulaire présente une longueur comprise entre 10 et 25 cm.

## Patentansprüche

1. Harnleiterstent (2), der einen Nierenendteil (200) und einen Blasenendteil (210) umfasst, wobei der Nierenendteil (200) röhrenförmig und gebogen ist,
wobei der Stent (2) **dadurch gekennzeichnet ist, dass** der Blasenendteil (210) mindestens einen Draht umfasst, und dadurch, dass der Stent ferner einen röhrenförmigen Zwischenabschnitt (220) umfasst, der sich zwischen dem Nierenendteil und dem Blasenendteil erstreckt, wobei der Abschnitt einen in Richtung des Blasenendteils abnehmenden Außendurchmesser (D₂₂₀) aufweist, und der Mindestaußendurchmesser des Abschnitts (220) im Bereich der Verbindung mit dem Draht des Blasenendteils (210) kleiner ist als das Zweifache des Durchmessers des Drahtes oder der summierte Querschnitt der Drähte, wenn mehrere davon vorhanden sind, und auf jeden Fall strikt kleiner als 1 mm ist, wobei der Zwischenabschnitt (220) eine Länge aufweist, die zwischen 0.5 cm und 12 cm enthalten ist.

2. Harnleiterstent (2) nach Anspruch 1, wobei der oder die Drähte einen kreisförmigen Durchmesser aufweisen, wobei jeder Draht einen Durchmesser (D₂₁₀) aufweist, der kleiner als 0,15 mm ist.

3. Harnleiterstent (2) nach Anspruch 1 oder 2, wobei der Blasenendteil (210) einen einzigen Draht umfasst.

4. Harnleiterstent nach einem der vorhergehenden Ansprüche, wobei der Zwischenabschnitt (220) eine Länge von 3 cm aufweist.

5. Harnleiterstent nach einem der vorhergehenden Ansprüche, wobei der Durchmesser des Zwischenabschnitts (220) über die gesamte Länge des Abschnitts auf regelmäßige Art und Weise abnimmt.

6. Harnleiterstent (2) nach einem der vorhergehenden Ansprüche, wobei der Zwischenabschnitt (220) abgeschrägt ist.

7. Harnleiterstent (2) nach einem der Ansprüche 3 bis 6, wobei der Zwischenabschnitt (220) einen röhrenförmigen Abschnitt (224) und einen massiven Abschnitt (225) umfasst, der durch den Blasenendabschnitt (210) verlängert ist, wobei der Draht des Blasenendabschnitts (210) aus einem Material mit einer Wand des Zwischenabschnitts (220) hergestellt ist.

8. Harnleiterstent (2) nach einem der vorhergehenden Ansprüche, wobei der Nierenendteil (210) eine Länge aufweist, die größer oder gleich 2 cm ist und vorzugsweise zwischen 5 und 30 cm enthalten ist.

9. Harnleiterstent (2) nach einem der Ansprüche 1 bis 8, wobei der Nierenendteil (200) und der Zwischenabschnitt (220) jeweils mehrere durchgehende Bohrungen (203, 223) umfassen.

10. Harnleiterstent (2) nach einem der Ansprüche 1 bis 9, der ferner einen röhrenförmigen Harnleiterteil (240) umfasst, der zwischen dem Nierenendteil (200) und dem Zwischenabschnitt (220) angeordnet ist, wobei der Harnleiterteil (240) einen Außendurchmesser (D₂₄₀) aufweist, der gleich demjenigen des Nierenendteils (200) ist und keine Bohrungen aufweist.

11. Harnleiterstent (2) nach dem vorhergehenden Anspruch, wobei der röhrenförmige Harnleiterteil (240) eine Länge aufweist, die zwischen 10 und 25 cm enthalten ist.

## Claims

1. An endo-ureteral stent (2), comprising a kidney end part (200) and a bladder end part (210), the kidney end part (200) being tubular and curved,
said stent (2) being **characterized in that** the bladder end part (210) includes at least one thread, and **in that** the stent further comprises a tubular intermediate portion (220) extending between the kidney end part and the bladder end part, said portion having an external diameter (D₂₂₀) decreasing toward the bladder end part, and the minimum external diameter of said portion (220), at the connection with the thread of the bladder end part (210) is less than twice the diameter of the thread, or the cumulative section of the threads if there are several of them, and in all cases must be strictly less than 1 mm, said intermediate portion (220) having a length between 0.5 cm and 12 cm.

2. The endo-ureteral stent (2) according to claim 1, wherein the thread(s) is/are of circular section, each thread having a diameter (D₂₁₀) of less than 0.15 mm.

3. The endo-ureteral stent (2) according to claim 1 or 2, wherein the bladder end part (210) includes a single thread.

4. The endo-ureteral stent according to one of the preceding claims, wherein the intermediate portion (220) has a length of 3 cm.

5. The endo-ureteral stent according to one of the preceding claims, wherein the diameter of the intermediate portion (220) decreases evenly over the whole length of said portion.

6. The endo-ureteral stent (2) according to one of the preceding claims, wherein the intermediate portion (220) is beveled.

7. The endo-ureteral stent (2) according to one of claims 3 to 6, wherein the intermediate portion (220) comprises a tubular portion (224) and a solid portion (225) extended by the bladder end portion (210), the thread of the bladder end portion (210) being integrally formed with a wall of the intermediate portion (220).

8. The endo-ureteral stent (2) according to one of the preceding claims, wherein the bladder end part (210) has a length greater than or equal to 2 cm, preferably between 5 and 30 cm.

9. The endo-ureteral stent (2) according to one of claims 1 to 8, wherein the kidney end part (200) and the intermediate portion (220) each comprise a plurality of through perforations (203, 223).

10. The endo-ureteral stent (2) according to one of claims 1 to 9, further comprises a tubular ureteral part (240) arranged between the kidney end part (200) and the intermediate portion (220), said ureteral part (240) having an external diameter (D₂₄₀) equal to that of the kidney end part (200) and being unperforated.

11. The endo-ureteral stent (2) according to the preceding claim, wherein the tubular ureteral part (240) has a length between 10 and 25 cm.
